# EUROPEAN PATENT APPLICATION

(11) **EP 2 790 120 A1**
(43) Date of publication of application: **15.10.2014**
(21) Application number: 14164359.3
(22) Date of filing: 11.04.2014
(51) Int. Cl.: G06F 19/00

(54) **Medical care information display control apparatus, medical care information display control method, and medical care information display control program**

(30) Priority: 12.04.2013 JP 2013083436
(71) Applicant: Fujifilm Corporation, Minato-ku Tokyo (JP)
(72) Inventor: Tanaka, Fumitake, Kanagawa-ken (JP); Kurami, Yoshiyuki, Kanagawa-ken (JP)
(74) Representative: Klunker . Schmitt-Nilson . Hirsch

(57) **Abstract**

[Problems to be Solved]

Properly determining, based on a number of display days of a region in which medical care information is displayed and a display reference date, display dates for the number of display days of the region in which medical care information is displayed and displaying the medical care information on a display screen.

[Means for Solving the Problems]

Identifying a display reference date according to a position in a first region (R2) representing a time axis received on a display screen, obtaining a number of display days of a second region (R7) in which a plurality of sets of medical care information of a display target person is displayed in time series on a daily basis, determining a plurality of medical care data dates as a plurality of display dates to be displayed in the second region (R7) in the order of closeness to the display reference date in a time axis direction such that the number of display days is satisfied based on the plurality of medical care data dates and the number of display days, and displaying medical care information corresponding to the determined plurality of display dates in the second region (R7) on a display screen.

## Description

### BACKGROUND OF THE INVENTION

### Field of the Invention

The present invention relates to a medical care information display control apparatus, medical care information display control method, and medical care information display control program for displaying a plurality of sets of diagnostic information of a display target person in time series.

### Description of the Related Art

Recently, in the field of medicine, a plurality of sets of medical care information, such as the results of examinations performed on a patient and the like, is displayed in time series and used as the reference information for diagnosing the disease name, determining treatment policy, and the like when a diagnosis is performed.

For example, Japanese Patent No. 5018463 proposes a method in which the presence or absence of medical care information in a preset time period is displayed in a first display region (main display region), then if a reference date is inputted in the main display region, a display period of a second display region (detail display region) is determined such that the total number of medical records displayed in the detail display region is equal to or greater than a threshold value based on the number of medical records present in the period before and after the reference date, and the number of records of medical care information is displayed in the detail display region in a bar chart along the time axis based on the determined display period. Further, Japanese Unexamined Patent Publication No. 2005-141531 proposes a method in which, in order to inspect the route of infection of bacteria test results, the bacteria test results are displayed in time series with respect to each region.

### SUMMARY OF THE INVENTION

According to the method of Japanese Patent No. 5018463, however, the time axis of the time series graph displayed in the detail display region is set associated with each of all dates included in the display period regardless of whether or not a medical record is present, so that the display space of the detail display region is not utilized efficiently. Further, according to the method of Japanese Unexamined Patent Publication No. 2005-141531, however, the medical care information is displayed in a predetermined period traced back from the latest inspection date, so that there has been a case in which medical care information of a particular inspection date desired by the user can not be displayed.

The present invention has been developed in view of the circumstances described above, and it is an object of the present invention to provide a medical care information display control apparatus, a medical care information display control method, and a medical care information display control program that perform display control in which display dates are properly determined based on a number of display days of a region in which medical care information is displayed and a display reference date for the number of display days of the region in which medical care information is displayed, and the medical care information is displayed on a display screen.

A medical care information display control apparatus according to the present invention includes:
a first region display control unit that displays a first region representing a time axis on a display screen;
a reference date identification unit that receives an input of a position in the displayed first region and identifies a display reference date according to the received position;
a medical care information obtaining unit that obtains a plurality of sets of medical care information of a display target person which includes a plurality of sets of medical care data associated with a plurality of medical care data dates respectively; and
a second region display control unit that obtains a number of display days of a second region in which the obtained plurality of sets of medical care information is displayed in time series on a daily basis, determines the plurality of medical care data dates as a plurality of display dates to be displayed in the second region in the order of closeness to the display reference date in a time axis direction such that the number of display days is satisfied based on the obtained plurality of medical care data dates and the number of display days, and displays the second region on a display screen based on the determined plurality of display dates and each set of medical care data corresponding to each display date.

A medical care information display control method according to the present invention operates a medical care information display control apparatus, the method including:
a first region display step that displays a first region representing a time axis on a display screen;
a reference date identification step that receives an input of a position in the displayed first region and identifies a display reference date according to the received position;
a medical care information obtaining step that obtains a plurality of sets of medical care information of a display target person which includes a plurality of sets of medical care data associated with a plurality of medical care data dates respectively; and
a second region display control step that obtains a number of display days of a second region in which the obtained plurality of sets of medical care information is displayed in time series on a daily basis, determines the plurality of medical care data dates as a plurality of display dates to be displayed in the second region in the order of closeness to the display reference date in a time axis direction such that the number of display days is satisfied based on the obtained plurality of medical care data dates and the number of display days, and displays the second region on a display screen based on the determined plurality of display dates and each set of medical care data corresponding to each display date.

A medical care information display control program according to the present invention causes a computer to function as:
a first region display control unit that displays a first region representing a time axis on a display screen;
a reference date identification unit that receives an input of a position in the displayed first region and identifies a display reference date according to the received position;
a medical care information obtaining unit that obtains a plurality of sets of medical care information of a display target person which includes a plurality of sets of medical care data associated with a plurality of medical care data dates respectively; and
a second region display control unit that obtains a number of display days of a second region in which the obtained plurality of sets of medical care information is displayed in time series on a daily basis, determines the plurality of medical care data dates as a plurality of display dates to be displayed in the second region in the order of closeness to the display reference date in a time axis direction such that the number of display days is satisfied based on the obtained plurality of medical care data dates and the number of display days, and displays the second region on a display screen based on the determined plurality of display dates and each set of medical care data corresponding to each display date.

The "plurality of sets of medical care information of a display target person which includes a plurality of sets of medical care data associated with a plurality of medical care data dates respectively" described above may be any medical care information as long as it includes medical care data which can be displayed on a daily basis and is referenced for diagnosis or treatment of the display target person. Further, the plurality of sets of medical care information may be formed of one type of medical care information which includes a plurality of items, each including medical care data which can be displayed on a daily basis or formed of a plurality of types of medical care information, each including medical care data which can be displayed on a daily basis with respect to one or more items.

Examples of the medical care information may include examination information, such as sample test information, image inspection information, and the like, treatment information, such as medication information and the like, diagnostic information, such as an electronic medical record which includes information of diagnosis recorded by a doctor, such as diagnosis, symptom, surgical treatment of the display target person and the like, and biological information of the patient, such as the body temperature, blood pressure, respiration rate, and the like of the display target person. The image inspection information may be any information as long as it is examination information related to an examination performed using an image of the display target person. The image inspection information may include, for example, images obtained by various types of modalities, such as ultrasonic diagnostic equipment, CT equipment, MRI equipment, CR equipment, and the like, and their interpretation reports.

The "medical care data date" may be the day when the medical care data were initially inputted (obtained) or the day when the medical care data were updated last. For example, in the case of image inspection information, the medical care data date may be the day when the image was obtained, in the case of sample test information, the medical care data date may be the day when the sample test was conducted, in the case of electronic medical record, the medical care data date may be the day when the electronic medical record was generated or the day when it was updated last, in the case of medication information, the medical care data date may be the day when the drug was administered, and in the case of biological information, the medical care data date may be the day when the biological information was measured.

In the medical care information display control apparatus according to the present invention, it is preferable that the medical care information obtaining unit obtains two or more types of medical care information arbitrarily selected from the group consisting of image inspection information, sample test information, medication information, and biological information.

Further, it is preferable that the medical care information display control apparatus further includes a display days determination unit that determines the number of display days according to the size of the second region, such that the greater the size of the second region, the greater the number of display days.

Still further, in the medical care information display control apparatus, it is preferable that the plurality of display dates of the second region is identifiably displayed in the first region. For example, it is preferable that the first region display control unit identifiably displays an index that indicates the plurality of display dates of the second region in the first region.

In the aforementioned case, the first region display control unit may identifiably display an index that indicates a display period of the second region with the oldest date of the plurality of display dates as the start date and the latest date of the plurality of display dates as the end date in the first region.

For the aforementioned "display dates", any index may be used as long as it is capable of identifying the display dates in the first region. For example, an index may be displayed at a position corresponding to each display date with respect to each of the display dates or an index that indicates a period with the oldest medical care data date of the display dates as the start date and the latest medical care data date of the display dates as the end date may be displayed. Further, an index of any shape may be used. For example, an index having a circular shape or a polygonal shape, such as a triangular shape, may be used. For example, a semitransparent rectangular index may be displayed at a position of the time axis of the first region corresponding to the display period.

Further, in the medical care information display control apparatus according to the present invention, the reference date identification unit may select, based on the received position and the position of the index that indicates the display period, any one of a first display date condition in which the plurality of display dates is determined only before the identified display reference date, a second display date condition in which the plurality of display dates is determined only after the display reference date, and a third display condition in which the plurality of display dates is determined both before and after the display reference date, and the second region display control unit may determine the plurality of display dates based on the selected one display date condition.

In the medical care information display control apparatus according to the present invention, it is preferable that the first region display control unit further displays an index that identifiably indicates presence or absence of the medical care data along the time axis in the first region.

For the "index that identifiably indicates presence or absence of the medical care data" described above, an index of any shape may be used as long as it allows presence or absence of medical care information to be identifiable. For example, an index having a circular shape or a polygonal shape, such as a triangular shape, a rectangular shape, or the like, may be displayed at a position corresponding to a medical care data date of medical care information. If medical care data dates of a plurality of sets of medical care information are present within a given period, one bar-like index that indicates the presence of the plurality of sets of medical care information may be displayed. Further, different types of indices may be used for different types or items of medical care information.

Further, in the medical care information display control apparatus according to the present invention, the second region display control unit may determine a number of a plurality of medical care data dates corresponding to a given set of medical care information of the plurality of sets of medical care information in the order of closeness to the display reference date in time series such that the number of the plurality of medical care data dates is the number of display days, and display the second region on a display screen based on the determined plurality of display dates and medical care data corresponding to the display dates respectively.

According to the present invention, a first region representing a time axis is displayed, an input of a position in the displayed first region is received and a display reference date is identified according to the received position, a plurality of sets of medical care information of a display target person which includes a plurality of sets of medical care data associated with a plurality of medical care data dates respectively is obtained, a number of display days of a second region is obtained in which the obtained plurality of sets of medical care information is displayed in time series on a daily basis, the plurality of medical care data dates is determined as a plurality of display dates to be displayed in the second region in the order of closeness to the display reference date along the time axis such that the number of display days is satisfied based on the obtained plurality of medical care data dates and the number of display days, and the second region is displayed on a display screen based on the determined plurality of display dates and each set of medical care data corresponding to each display date. Consequently, a plurality of display dates may be identified for the number of display days of the second region in the order of closeness to the display reference date along the time axis such that a date without medical care data is not included in the display dates. This allows a plurality of sets of medical care information to be displayed through efficient use of display space of the second region in comparison with the case in which a plurality of display dates is identified to include a date without medical care data.

### BRIEF DESCRIPTION OF THE DRAWINGS

Figure 1 illustrates a schematic configuration of a medical information system to which a medical care information display control apparatus according to a first embodiment is applied.
Figure 2 illustrates an example medical care information display screen.
Figure 3 is a flowchart illustrating a medical care information display control flow in the first embodiment.
Figure 4 is a drawing for explaining a preferable example of table data in a second embodiment.

### DESCRIPTION OF THE PREFERRED EMBODIMENTS

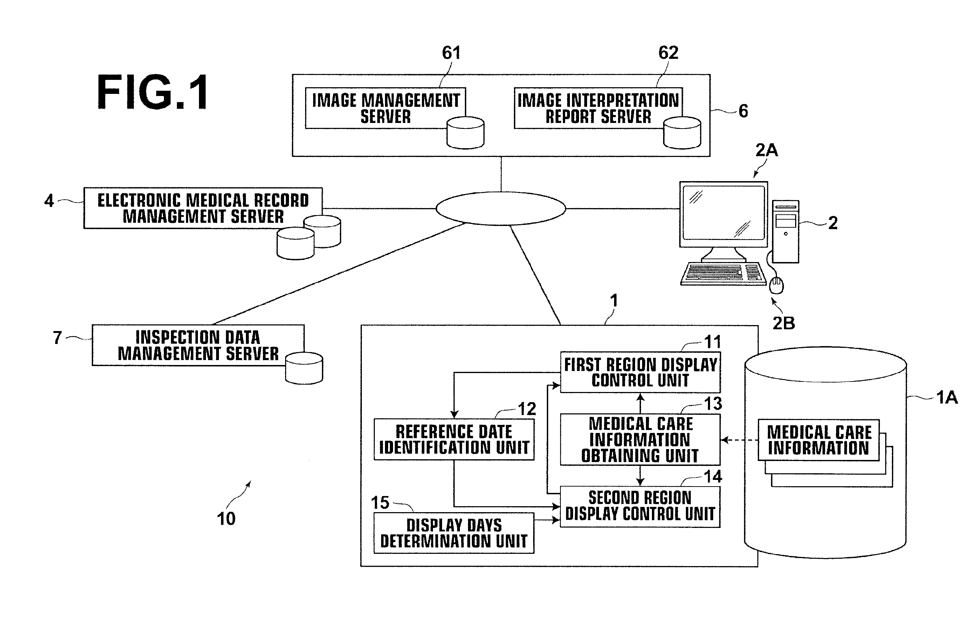

Hereinafter, an embodiment of the medical care information display control apparatus of the present invention will be described based on Figure 1. Figure 1 illustrates a schematic configuration of a medical information system to which a medical care information display control apparatus according to a first embodiment of the present invention is applied. It is a functional block diagram of the medical care information display control apparatus of an embodiment of the present invention.

As illustrated in Figure 1, a medical information system 10 includes a medical care information display control apparatus 1, a diagnosis and treatment department terminal 2, an electronic medical record management server 4, an image diagnosis system 6, and an inspection data management server 7 which are communicatively formed via a network.

The electronic medical record management server 4 is a computer provided with an electronic medical record database which stores electronic medical records, and includes software for retrieving medical care information, such as an image associated with each electronic medical record, inspection results, or the like, and performing transmission/reception of retrieval results in response to a request from the diagnosis and treatment department terminal 2 or the like, in addition to an operating system and database management software. The electronic medical record management server 4 is connected to the medical care information display control apparatus 1, the diagnosis and treatment department terminal 2, the inspection data management server 7, the image diagnosis system 6, and the like via a network so as to be able to obtain each set of medical care information associated with electronic medical records.

The inspection data management server 7 is a computer provided with an inspection data management database which stores inspection data, and includes inspection data management software in addition to standard software, such as an operating system. Sample test information that includes test date of a sample test performed in the inspection room according to an inspection order inputted from each diagnosis and treatment department terminal 2, and test result data are inputted associated with the inspection order and the patient ID from an inspection room terminal (not shown) provided in each inspection room and stored in the inspection data management database via a network.

The image diagnosis system 6 is a known computer system. Here, it is of a configuration in which a workstation (not shown) for image diagnostic doctors, modalities (not shown), such as CT, MRI, and the like, an image management server 61 provided with an image database which stores image data obtained by the modalities, such as CT, MRI, and the like, and an image interpretation report server 62 provided with an image interpretation report database which stores image interpretation reports, including image interpretation results of the images obtained by the imaging, are communicatively connected via a network. The workstation for image diagnostic doctors includes various types of image analysis software and is configured so as to be able to perform various types of image analysis processing according to the purpose and target of the diagnosis.

The diagnosis and treatment department terminal 2 is a computer used by the doctors of diagnosis and treatment department and the like for viewing clinical information of a patient and inputting an inspection order, and the like, and includes a display unit 2A which is a common display and an input unit 2B which includes a keyboard and a mouse. The diagnosis and treatment department terminal 2 is also used for displaying result data of an inspection performed in each diagnosis and treatment department or medical care information, such as a generated electronic medical record, for reference. The diagnosis and treatment department terminal 2 includes standard software, such as an operation system, and application software for displaying medical care information, such as a generated electronic medical record.

In the present embodiment, when an instruction operation to start displaymedical care information and a specification (or input) operation of necessary information, such as the patient ID, made by a user, such as a doctor, are received by the input unit 2B, such as a mouse or a keyboard, of the diagnosis and treatment department terminal 2, and various instructions for displaying medical care information and required information according to the various instructions are inputted by the input unit 2B, the diagnosis and treatment department terminal 2 transmits the various instructions and data corresponding to the various instructions, as required, to the medical care information display control apparatus 1, to be described later. The medical care information display control apparatus 1 receives these various instructions (and corresponding data, as required) and transmits information required to display medical care information of the present embodiment, such as display setting information that defines the display setting and a plurality of sets of medical care information corresponding to the inputted patient ID, and the like, to the diagnosis and treatment department terminal 2. The, the diagnosis and treatment department terminal 2 receives the display setting information and required information, and displays a medical care information display screen, to be described later, on the display screen of the display unit 2A based on the received display setting information and required information.

The medical care information display control apparatus 1 is a computer provided with a medical care information management database 1A. Further, the medical care information display control apparatus 1 includes an operating system and database management software and functions also as a medical information management server. The medical care information display control apparatus 1 is connected to the electronic medical record management server 4, the diagnosis and treatment department terminal 2, the inspection data management server 7, the image management server 61, and the image interpretation report server 62 via a network, and retrieves and obtains medical care information of a patient, such as the electronic medical record of the patient, various inspection result data, image data, image interpretation report, and the like, from each of the connected servers or the like based on the patient ID, and stores the medical care information in the medical care information management server 1A after associating the information with the patient ID. In the medical care information, the each set of medical care information included in the medical care information is associated with each medical data date and stored. The medical care information display control apparatus 1 updates the medical care information to be managed at a fixed time every day. Further, the medical care information display control apparatus 1 receives, as appropriate, medical care information transmitted from each of the servers or the like and updates the medical care information to be managed in response to a request from each of the servers or the like. Still further, the medical care information display control apparatus 1 obtains medical care information from each of the servers or the like and updates the medical care information to be managed as required by the medical care information display control apparatus 1.

The medical care information display control apparatus 1 of the present embodiment includes a medical care information display control program according to the present embodiment. By the execution of the medical care information display control program, the medical care information display control apparatus 1 functions as a first region display control unit 11 that displays a first region R2 representing a time axis on the display screen, a reference date identification unit 12 that receives an input of a position in the displayed first region and identifies a display reference date according to the received position, a medical care information obtaining unit 13 that obtains a plurality of sets of medical care information of a display target person which includes a plurality of sets of medical care data associated with a plurality of medical care data dates respectively, a display days determination unit 15 that determines a number of display days of a second region R7 which is a region in which the obtained plurality of sets of medical care information is displayed in time series on a daily basis, and a second region display control unit 14 that obtains a number of display days of the second region, determines the plurality of medical care data dates as a plurality of display dates to be displayed in the second region in the order of closeness to the display reference date in a time axis direction such that the number of display days is satisfied based on the obtained plurality of medical care data dates and the number of display days, and displays the second region on the display screen based on the determined plurality of display dates and each set of medical care data corresponding to each display date.

The medical care information management database 1A of the medical care information display control apparatus 1 also includes display setting information required to display a plurality of sets of medical care information on the diagnosis and treatment department terminal 2 in a predetermined display format.

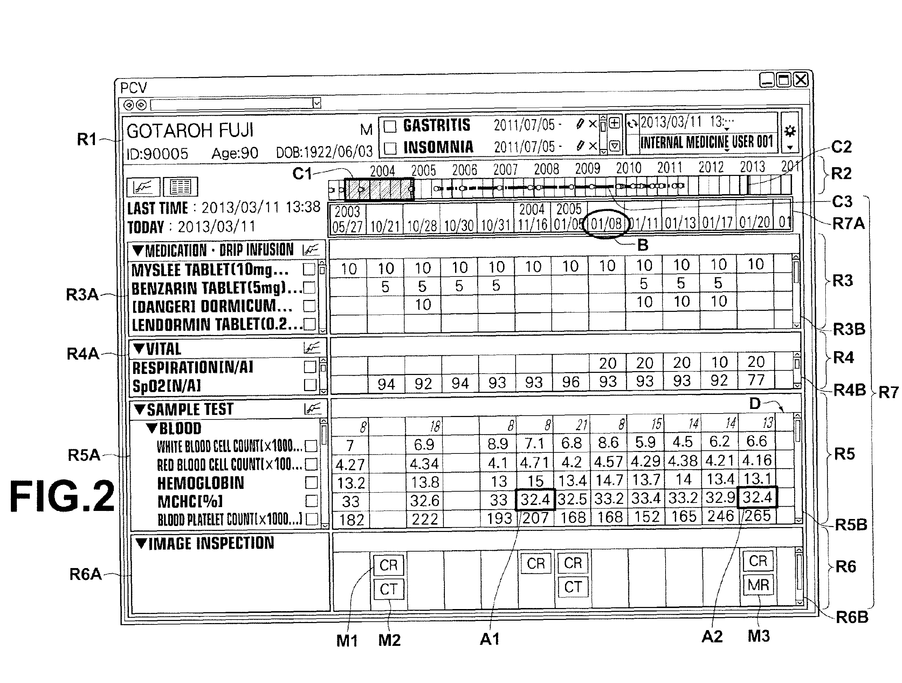

A medical care information display screen display controlled by the medical care information display control apparatus 1 will now be described. Figure 2 illustrates an example medical care information display screen.

As shown in Figure 2, the medical care information display screen includes a basic information display section R1, a first region R2 which is a display section that indicates a time axis according to the period in which medical care records of a patient is present, and a second region R7 which is a region in which a plurality of sets of medical care information of a patient is displayed in time series on a daily basis.

In the present embodiment, medical care data of a patient are displayed in the second region R7 in a table format, as shown in Figure 2, based on table data, to be described, generated by the second region display control unit 14. More specifically, the second region R7 includes a display date section R7A that indicates display dates of second region R7 in time series, a medication information display section R3 for displaying various types of medical care information, a biological information display section R4, a sample test information display section R5, and an image inspection information display section R6. As shown in Figure 2, only the dates on which medical care data are present, which are determined as display dates, to be described later, are displayed in time series in the display date section R7A.

The basic information display section R1 is a section in which basic information is displayed, such as the identification information of a patient (patient ID), name, age, gender, past medical history, and the like.

The first region R2 is a region which represents a time axis. As shown in Figure 2, the first region R2 indicates the time axis with the numbers representing years and scales. Here, the time axis is set to include the period in which medical care data of a patient are present and a predetermined additional period. If the period in which medical care data of a patient are present is shorter than a predetermined period as in a new patient or the like, the time axis is set so as to indicate a predetermined initial period. Further, a red line C2 representing the current date is displayed at a position of the first region R2 corresponding to the current date.

Still further, a rectangular index C1 that indicates the display period of the second region R7 is displayed in the first region R2. Here, the position on the past side end of the index C1 in a time axis direction indicates the leftmost display date displayed in the display date section R7A of the second region R7 in Figure 2 and the position on the future side end of the index C1 in the time axis direction indicates the rightmost display date displayed in the display date section R7A of the second region R7 in Figure 2. Note that the rectangular index C1 that indicates the display period of the second region R7 is semitransparent in order to secure visibility of the other indices and the time axis (scales, here) displayed in the first region R2.

Further, rectangular indices C3 that indicate the presence or absence of medical care data along the time axis are displayed at the positions corresponding to the medical care data dates of the medical care data in the first region R2. In the present embodiment, a plurality of black rectangular indices C3 is displayed according to the dates on which medical care data are present, and the plurality of indices C3 is indicated as an integrated bar extending in a time axis direction in the portion where the dates on which medical care data are present appear successively in close proximity to each other. The circular index indicates the date of admission and the white square index indicates the date of discharge.

The medication information display section R3 is a section that displays information of drug administration to the patient and includes a medical item list display section R3A and a medication data display section R3B that displays dosage data. The medication data display section R3B numerically displays data of the dose of administered drug with respect to each drug included in the item list of the medication information according to the drug administration date (medical care data date).

The biological information display section R4 is a section that displays biological information of the patient and includes a biological information item list display section R4A and a biological data display section R4B. The biological data display section R4B numerically displays biological data of the patient with respect to each item included in the item list of the biological information of the patient according to the biological information measurement data (medical care data date). The data indicating an abnormal value in the biological data are identifiably displayed in red.

The sample test information display section R5 is a section that displays test result data of a plurality of sample tests and includes a sample test item list display section R5A and a test result data display section R5B. The test result data display section R5B numerically displays test result data with respect to each test item included in the item list of the sample test information according to each test date of the test result data (medical care data date). In Figure 2, note that the numerical values enclosed by thick frames A1 and A2 are identifiably displayed in red.

The image inspection information display section R6 is a section that displays image inspection information of the patient and the presence or absence of image inspection results for the respective types of image inspection (CR, CT, MR) are displayed by predetermined, identifiable icons M1, M2, M3 with respect to each inspection date of image inspection (medical care data date). If the icon M1, M2, or M3 is selected by a mouse click or the like, a diagnostic image corresponding to the type of the selected icon and corresponding to the corresponding medical care data is displayed on a separate display screen together with the image interpretation report of the diagnostic image. Further, if the icon M1, M2, or M3 is selected by an operation, such as mouseover or the like, a thumbnail image of the diagnostic image corresponding to the type of the selected icon and corresponding to the corresponding medical care data is displayed.

The vertical and horizontal sizes of the columns of the table data displayed in the data display sections are predetermined sizes. In the case where the size of the data display section does not become an integer multiple of the predetermined size of the column, each column is displayed in a displayable range of the data display section, as illustrated in the arrow D in Figure 2.

Further, a scroll bar for changing the display range in up-down directions is provided in each of the item list display sections (R3A, R4A, R5A, and R6A in Figure 2) and in each of the data display sections (R3B, R4B, R5B, and R6B in Figure 2). Still further, a check box is displayed for each item of the list in each of the item list display sections (R3A, R4A, R5A, and R6A in Figure 2).

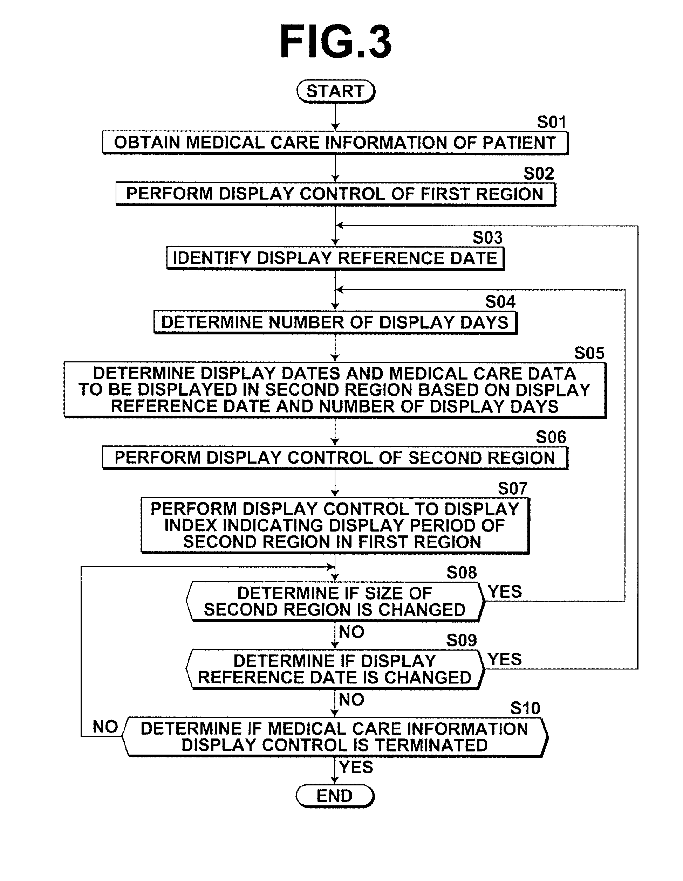

A flowchart that describes a processing flow of the medical care information display control apparatus 1 according to the first embodiment is shown in Figure 3. A processing flow of the medical care information display control apparatus 1 will be described according to Figure 3.

First, in the diagnosis and treatment department terminal 2, when an instruction to display medical care information according to the present embodiment and a patient ID of a patient who is the display target person are inputted from the input unit 2B based on an operation by a user, such as a doctor or the like, the diagnosis and treatment department terminal 2 transmits the inputted patient ID and the medical care information display start instruction to the medical care information display control apparatus 1. Then, the medical care information display control apparatus 1 receives the patient ID and the medical care information display start instruction and performs the medical care information display processing according to the present embodiment.

First, the medical care information obtaining unit 13 obtains the patient ID transmitted from the diagnosis and treatment department terminal 2 and obtains a plurality of sets of medical care information from the medical care information management database 1A (S01). The medical care information display control apparatus 1 transmits an instruction to display the basic information display section R1 and information necessary for the display to the diagnosis and treatment terminal 2 based on display setting information for the display of the medical care information and the obtained plurality of sets of medical care information. Then, based on the display setting information, a medical care information display screen is displayed on the display unit 2A of the diagnosis and treatment department terminal 2 and basic information of the patient is displayed in the basic information display section R1.

In each embodiment described in the present specification, the medical care information obtaining unit 13 obtains, based on the patient ID, all sets of medical care information related to the patient ID from the medical care information management database 1A. Here, it is assumed that diagnostic information, such as an electronic medical record, image inspection information, sample test information, biological information, and treatment information have been obtained.

Then, based on the obtained plurality of sets of medical care information, the first region display control unit 11 obtains the medical care data date of each set of medical care data included in the plurality of sets of medical care information and calculates an entire medical care period with the oldest date of all of the obtained medical care data dates as the start date and the latest date of all of the obtained medical care data date as the end date. Then, a comparison is made between the oldest date of the entire medical care period and the date obtained by subtracting a predetermined past display period (about one year) from the current date and an older date is determined as the start date of the period represented by the time axis of the first region R2. Then, the date obtained by adding a predetermined future display period (about one year) to the future side of the time axis direction of the entire medical care period is determined as the end date of the period represented by the time axis of the first region R2 (display period of the first region R2).

Then, the first region display control unit 11 performs display control to display the first region R2 in a predetermined display format according to the display period of the first region R2 (S02). Here, the first region display control unit 11 transmits an instruction to display the first region and necessary information, such as the display period and the like, to the diagnosis and treatment department terminal 2. Then, the first region R2 is displayed on the display screen of the diagnosis and treatment department terminal 2.

Further, in the present embodiment, the first region display control unit 11 performs display control of the diagnosis and treatment department terminal 2 to display a rectangular index C3, which indicates the presence of medical care data of the patient, at the medical care data date position corresponding to the medical care data in the first region R2.

Further, if the display period of the second region R7 is set, the first region display control unit 11 performs display control of the diagnosis and treatment department terminal 2 to display a rectangular index C1 representing the display period of the second region R7 at the corresponding position on the time axis of the first region R2. In the mean time, if the display period of the second region R7 is not set, such as the case of blank display of the second region R7, the first region display control unit 11 performs display control of the diagnosis and treatment department terminal 2 to display a rectangular index C1 representing the display period of the second region R7 at a predetermined position of the first region R2.

Next, a position in the first region R2 is inputted by the input unit 2B based on a user operation, the diagnosis and treatment department terminal 2 receives the inputted position and transmits the position to the medical care information display control apparatus 1. Then, based on the inputted position and the relative position of the time axis of the first region R2, the reference date identification unit 12 identifies the display reference date corresponding to the inputted position (S03).

In the present embodiment, the reference date identification unit 12 further obtains a display position of the rectangular index C1 representing the display period of the second region R7 in the first display region R2. Then, if the inputted position corresponds to the most future side end of the rectangular index C1 representing the display period of the second region R7 in the time axis direction, the reference date identification unit 12 selects a first display date condition in which a plurality of display dates is determined only before the identified display reference date. If the inputted position corresponds to the most past side end of the rectangular index C1 representing the display period of the second region R7 in the time axis direction, the reference date identification unit 12 selects a second display date condition in which a plurality of display dates is determined only after the display reference date. Further, if the inputted date does not correspond to the ends of the index C1 representing the display period of the second region R7 in the time axis direction, the reference date identification unit 12 selects a third display date condition in which a plurality of display dates is determined both before and after the display reference date. Note that if the most past side end (or most future side end) of the rectangular index C1 representing the display period of the second region R7 in the time axis direction is dragged, the reference date identification unit 12 deems the position of the dragged end point as the inputted position.

In the example of Figure 2, the third display date condition is selected and January 8, 2005 is identified as the display reference date (ellipse B). If the reference date identification unit 12 is unable to identify the position inputted to the first region R2, such as the case where a user operation for inputting a position to the first region R2 is not performed at the diagnosis and treatment department terminal 2 or the like, the reference date identification unit 12 identifies the current date as the reference date and selects the first display date condition.

Next, the display days determination unit 15 obtains the size of the second region R7 from the diagnosis and treatment department terminal 2. The medical care information management database 1A includes display days determination information in which the number of display days is associated with each width of the second region R7 in a time axis direction, such that the greater the size of the second region R7 in the time axis direction, the greater the number of display days. The display days determination unit 15 determines the number of display days to be displayed in the second region R7 according to the width of the second region R7 based on the display days determination information (S04). The number of display days to be displayed is preset according to the size of the second region R7, such that each column of the second region R7 that displays the medical care data becomes an appropriate size for visibility.

The second region display control unit 14 obtains the determined number of display days and retrieves the medical care data date of each set of medical care information in the past and future directions from the display reference date along the time axis. Then, the second region display control unit 14 determines medical care data dates as a plurality of display dates in the order of closeness to the display reference date in a time axis direction such that the number of display days is satisfied, and obtains each set of medical care data corresponding to each display date (S05). This determines the display dates to be displayed in the second region R7 so as not to include a date without medical care data date of medical care data (date without medical care data) with respect to any of a plurality of sets of medical care information.

Then, based on the determined display dates and the obtained plurality of sets of medical care information, the second region display control unit 14 generates table data for displaying in each of the data display sections of the second region R7(refer to R3B, R4B, R5B, and R6B in Figure 2) . Based on each display date and each item of medical care information, each corresponding set of the medical care data is assigned to each section in the table data.

Further, as the third display date condition is selected in the present embodiment, a plurality of display dates is determined by retrieving medical care data dates in the past and future directions from the display reference date. Note that, if the first display date condition is selected, the second region display control unit 14 retrieves the medical care data dates of medical care information along the time axis only on the past side from the display reference date and determines a plurality of display dates in the manner as described above. If the second display date condition is selected, the second region display control unit 14 retrieves the medical care data dates of medical care information along the time axis only on the future side from the display reference date and determines a plurality of display dates in the manner as described above.

Then, based on the generated table data, the second region display control unit 14 performs display control to display the second region R7 (S06). More specifically, the second region display control unit 14 transmits a display instruction to display the second region R7 and data required for displaying the second region R7, such as the generated table data and the like, to the diagnosis and treatment department terminal 2, and the diagnosis and treatment department terminal 2 receives these and displays the second region R7 on the display unit 2A. Then, the second region R7 is displayed as in the display screen example shown in Figure 2.

The first region display control unit 11 obtains the display period of the second region R7 and updates the index C1 in the first region R2 indicating the display period of the second region R7 according to the display period of the second region R7 by display controlling the diagnosis and treatment department terminal 2 (S07) .

If an input by the user for changing the size of the second region R7 is received, the diagnosis and treatment department terminal 2 transmits the inputted size of the second region R7 to the display days determination unit 15 of the medical care information display control apparatus 1 (S08: Y) . Then, the display days determination unit 15 receives the size change of the second region R7 and determines the number of display days of the second region R7 for update according to the changed size of the second region R7 (S04). Then, based on the updated number of display days, the second region display control unit 14 performs processing steps from S05 to S07 as in the manner described above.

If the size of the second region is not changed (S08: N), reference date identification unit 12 checks to see if the display reference date is changed (S09). Here, if a user input of a further position in the first region R2 is received, the diagnosis and treatment department terminal 2 transmits the inputted position to the reference date identification unit 12 of the medical care information display control apparatus 1. Then, the reference date identification unit 12 judges that the display reference date is changed (S09: Y) and identifies the display reference date of the second region for update based on the inputted further position (S03). Then, the display days determination unit 15 performs the processing step S04 as in the manner described above, and the second region display control unit 14 performs the processing steps from S05 to S08 as in the manner described above based on the updated display reference date.

Further, if the size of the second region R7 is not changed (S09: N), the medical care information display control apparatus 1 checks the diagnosis and treatment department terminal 2 to see if an instruction to end the medical care information display processing is issued and if it is not issued (S10: N), repeats the processing steps S08 and S09. If a user input to end the medical care information display processing is received (S10: Y), the diagnosis and treatment department terminal 2 transmits the end instruction of the medical care information display processing to the medical care information display control apparatus 1. Then, the medical care information display control apparatus 1 ends the medical care information display processing.

According to the aforementioned first embodiment, a plurality of display dates may be identified for the number of display days of the second region R7 in the order of closeness to the display reference date along the time axis such that a date without medical care data is not included in the display dates. This allows a plurality of sets of medical care information to be displayed through efficient use of display space of the second region R7 in comparison with the case in which a plurality of display dates is identified to include a date without medical care data.

Further, in the case where the medical care information obtaining unit 13 obtains two or more types of medical care information arbitrarily selected from the group consisting of image inspection information, sample test information, medication information, and biological information, as in the first embodiment described above, a plurality of types of medical care data of a patient may be displayed in the second region R7. This allows the user, such as a doctor or the like, to make a comparative assessment of a plurality of types of medical care data easily, which may support the improvement in the medical accuracy and efficiency. In order to further enhance the advantageous effect, it is preferable that the plurality of sets of displayed medical care information includes treatment information, such as medication information and the like, and examination information, such as sample test information, image inspection information, and the like.

Further, if the medical care information display control apparatus 1 includes the display days determination unit 15 that determines the number of display days according to the size of the second region R7 such that the greater the size of the second region R7, the greater the number of display days, as in the first embodiment described above, the number of display days is determined so as to be appropriate for the size of the second region R7. This allows the user, such as a doctor or the like, to easily confirm the medical care data in the second region R7.

Still further, as in the first embodiment described above, it is preferable that the input unit 2B inputs a size change of the second region R7 on the medical care information display screen based on a user operation and transmits the size change to the medical care information display control apparatus 1, the display days determination unit 15 of the medical care information display control apparatus 1 obtains the size change of the second region R7 and changes and determines the number of display days according to the changed size, and the second region display control unit 14 newly performs display control of the second region R7 based on the determined new number of display days. Even when the size of the second region R7 is changed by the user, the user is not required to manually reset the number of display days and may confirm medical care data and the like in the second region R7 based on an appropriate number of display days.

Further, it is preferable that the first region display control unit 11 identifiably displays an index that indicates a plurality of display dates of the second region R7 in the first region R2, as in the first embodiment described above. This helps the user to intuitively understand the relationship between the display period of the first region R2 and the display dates of the second region R7. Further, by referencing to the index that indicates individual display dates, it becomes easy for the user to specify a position according to the time axis in the first region R2 in order to determine a new reference date.

Still further, if the first region display control unit 11 displays the index C1 that indicates the display period of the second region with the oldest date of a plurality of display dates as the start date and the latest date of the plurality of display dates as the end date at the corresponding position on the time axis of the first region R2, as in the first embodiment described above, it becomes easier for the user to intuitively understand the relationship between the time axis of the first region R2 and the display dates of the second region R7.

Further, in the case described above, if the first region display control unit 11 further displays the index C3 that identifiably indicates presence or absence of medical care data in the first region R2, it becomes easy for the user to specify the position for determining a new reference date in the first region R2.

Still further, as in the first embodiment described above, if the first region display control unit 11 displays both the index C1 that indicates the display period of the second region and the index C3 that indicates presence or absence of medical care information in the first region R2, it becomes easy for the user to specify the position for determining a new reference date in the first region R2 by referencing both the presence or absence of medical care data and the display period of the second region R7.

Further, it is preferable that the first region R2 and the second region R7 are displayed close to each other at positions where the first region R2 and the second region R7 are visually recognizable at a glance, as in the first embodiment described above. In this case, while displaying medical care information of the patient in the second region R7 and observing desired medical care data in detail, it is easy to briefly confirm the medical history of the patient by easily understanding the correspondence relationship between the second region R7 and the time axis of the first region R2 and visually recognizing the presence or absence of medical care information of the patient globally by the first region R2. Consequently, the improvement in the diagnostic efficiency and accuracy of the doctors and the like may be supported.

Still further, it is preferable that the number of display days is determined according to the size of the second region R7 such that a predetermined column size that allows easy visual recognition of the column by the user is secured. While efficiently utilizing the display space of the second region R7 by determining the display dates of the second region R7 by excluding a date without medical care data, the doctor or the like may confirm the medical care data in each column having a visually recognizable size.

Further, as in the first embodiment described above, it is preferable that the reference date identification unit 12 selects any one of the first display date condition in which a plurality of display dates is determined only before the identified display reference date, the second display date condition in which a plurality of display dates is determined only after the display reference date, and the third display date condition in which a plurality of display dates is determined both before and after the display reference date, based on the received position and the position of the index that indicates the display period, and the second region display control unit determines a plurality of new display dates based on the one display date condition selected. In this case, the second region R7 may be displayed by determining the display dates of the second region R7 based on the display date condition desired by the user.

Still further, in the first embodiment, the reference date identification unit 12 selects the first display date condition if the inputted position corresponds to the most future side end of the rectangular index C1 that indicates the display period of the second region R7 in the time axis direction, the second display date condition if the inputted position corresponds to the most past side end of the rectangular index C1 that indicates the display period of the second region R7 in the time axis direction, and the third display date condition if the inputted position does not correspond to the ends of the rectangular index C1 that indicates the display period of the second region R7 in the time axis direction. This allows each display date condition to be selected by a simple operation of the user to specify (click or drag) the end of the index C1 that indicates the display period of the second region R7 in the time axis direction. This allows the second region R7 to be displayed after setting a display date condition desired by the user easily and appropriately.

Further, as a modification of the first embodiment described above, the second region display control unit 14 may perform the processing shown in S05 in the following manner. When a display reference date is identified, the second region display control unit 14 obtains a plurality of sets of medical care data and a medical care data date of each set of the medical care data included in the month to which the reference date belongs (unit period) based on the display reference date and inserts each set of medical care data in table data having columns corresponding to the predetermined number of days associated with the obtained plurality of medical care data dates in the order of closeness to the display reference date in time series. In this case, if the first display condition is selected, the second region display control unit 14 inserts medical care data in time series only on the past side from the newest column of the table data in the time axis direction. If the second display condition is selected, the second region display control unit 14 inserts medical care data in time series only on the future side from the oldest column of the table data in the time axis direction. If the third display condition is selected, the second region display control unit 14 inserts past side and future side medical care data from the middle column of the table data in the time axis direction in the order of closeness of the medical care data date to the reference date on the time axis.

Next, if there is any column in the table data in which medical care data are not inserted (uninserted column), the second region display control unit 14 determines a new month (new unit period), then obtains a plurality of sets of medical care data of the patient and medical care data date of each set of the medical care data with respect to the new month, and repeats the operation of inserting the medical care data in the table data associated with the obtained medical care data date. If the first display condition is selected, the second region display control unit 14 determines a new unit period on the past side so as to be continued in time series, if the second display condition is selected, the second region display control unit 14 determines a new unit period on the future side so as to be continued in time series, and if the third display condition is selected, the second region display control unit 14 determines either of the successive unit periods on the future and past sides in time series which is closer to the reference date as a new unit period. When no uninserted column remains as a result of this, a table data identical to that of the first embodiment described above is generated.

It should be understood that the present invention is not limited to each embodiment described in the present specification, and the second region display control unit 14 may use any method as long as a plurality of display dates determined based on a plurality of medical care data dates and the number of display days eventually satisfies the number of display days when counted in the order of closeness to the display reference date in a time axis direction.

As a further modification of the first embodiment described above, the second region display control unit 14 may determine a plurality of medical care data dates corresponding to a given set of medical care information of a plurality of sets of medical care information as the display dates of the second region in the order of closeness to the display reference date in time series such that the number of the plurality of medical care data dates of the given set of medical care information corresponds to the number of display days, and may display the second region based on the plurality of determined display dates and medical care data corresponding to the display dates. The term "given set of medical care information" as used herein refers to medical care information of one item belonging to a give type.

It is conceivable, for example, that for each medical care data dates (test dates) of only one item of sample test of a plurality of sets of medical care information, a plurality of medical care data dates is determined as the display dates of the second region in the order of closeness to the display reference date in time series, and only the medical care data of the plurality of sets of medical care information corresponding to the plurality of determined display dates may be displayed. In this case, the table data may be generated such that only the medical care data dates of the medical care data of the given medical care information which is of interest to the doctor, and displayed in the second region R7. This may respond to the user demand to understand only the desired information easily and efficiently.

As a second embodiment, in the case where a new display reference date is identified after table data (latest table data) are generated, the second region display control unit 14 may generate new table data corresponding to the new display reference date by obtaining a part of the latest table data in the memory. Hereinafter, the second embodiment will be described. The second embodiment differs from the first embodiment only in the processing step S06 performed by the second region display control unit 14 and the function of each component shown in Figure 1 is common to that of the first embodiment for the others. Therefore, only the point that differs from the first embodiment will be mainly described in the second embodiment and the description of the others common to the first embodiment will be omitted in order to avoid duplicated description.

First, in the case where the latest table data are not present, such as the case in which table data have not been generated, processing steps S01 to S08 shown in Figure 3 are performed in the second embodiment, as in the first embodiment. Next, if the display reference date is changed (refer to S09: Y), the reference date identification unit 12 receives the change in the display reference date and identifies a new display reference date, as in the first embodiment (refer to S03). Then, the display days determination unit 15 determines a new number of display days based on the new display reference date, as in the first embodiment (refer to S04).

Next, the second region display control unit 14 determines whether or not the new display reference date falls in a judgment period with the oldest display date of the latest table data as the start date and the latest date of the latest table data as the end date.

Then, if the new display reference date does not fall in the judgment period, the second region display control unit 14 determines display dates and medical care data corresponding to the display dates, as in S05 of the first embodiment, and generates new table data.

On the other hand, if the new display reference date falls in the judgment period, the second region display control unit 14 extracts, based on a plurality of new display dates, display dates of a plurality of display dates of the latest table data overlapping with the display dates (overlapping display dates), copies a table data portion of the latest table data in the memory corresponding to the overlapping display dates to generate a portion corresponding to the overlapping display dates of the new table data and, for display dates of the newly determined display dates other than the overlapping display dates, obtains medical care data corresponding to the display dates, thereby generating new table data.

Then, the second region display control unit 14 performs display control of the diagnosis and treatment department terminal 2 to update and display the second region R7 on the display unit 2A of the diagnosis and treatment department terminal 2 based on the new table data, as in the first embodiment (refer to S06) . Then, as in the first embodiment, the medical care information display control apparatus 1 of the second embodiment performs the successive processing steps of S07 to S10.

According to the second embodiment, the medical care data in the range of the latest table data in which display dates overlap may be used effectively when generating new table data and therefore the load of processing may be reduced.

Further, in this case, as the second embodiment, the number of display days of the table data in the first embodiment is preferable to be the number of display days displayed in the display date section R7A of the second region R7 with some extra days added thereto. Note that it is assumed that the number of display days of the table data corresponds to the number of days displayed in the display date section R7A of the screen in the first embodiment.

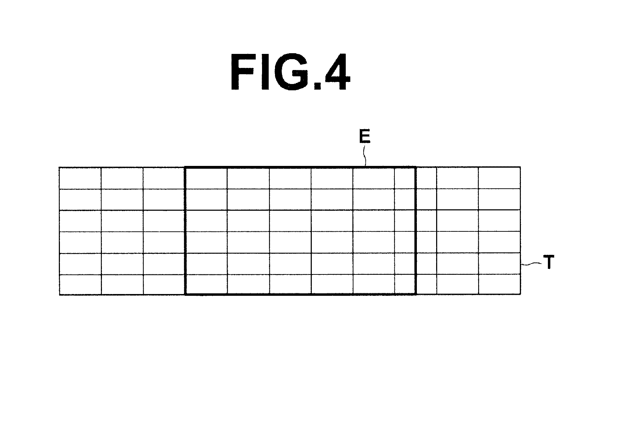

Figure 4 illustrates a concept of the table data according to the second embodiment. According to the second embodiment, the number of display days of the table data is the number of display days displayed in the display date section R7A of the second region R7 with some extra days added to the past and future sides thereof.

Consequently, as illustrated in Figure 4, the second region display control unit 14 generates table data T which include extra days as well as the thick frame portion E corresponding to the display dates displayed in the display date section R7A of the second region R7. Then, the second region display control unit 14 performs display control of the diagnosis and treatment department terminal 2 to display only the data corresponding to the thick frame portion E of the table data according to a given setting of the display setting information.

In the aforementioned case, the number of display days of the table data is the number of display days displayed in the display date section R7A of the second region R7 with some extra days added thereto. This may increase the possibility that display dates of the latest table data overlap with display dates of new table data, so that it is easy to obtain the effect of reducing the load of processing.

Further, in the second embodiment, for a portion of the table data corresponding to display dates other than the overlapping display dates, the table data generation method according to the modification of the first embodiment may be applied.

In each embodiment described above, the first region display control unit 11 may identifiably display the area of the time axis corresponding to the display period of the second region R7 by a different color from that of the other area, instead of using the index C1 that indicates the display period of the second region R7. Further, the first region display control unit 11 may identifiably display the positions corresponding to the medical care data dates of medical care data along the time axis of the first region by a color different from the other positions, instead of using the index C3 that indicates the presence or absence of medical care data. In the mean time, the first region display control unit 11 may display the time axis in any form, such as, in the form of a line, in the form of a bar, or the like, as long as it represents a time axis along a given direction. Still further, the first region display control unit 11 may omit the identification display of the presence or absence of medical care data in the first region R2. Further, the first region display control unit 11 may omit the identification display of display period of the second region R7 in the first region R2.

Further, the present invention is not limited to each of the embodiments described above, and a plurality of sets of medical care information to be displayed in the second region R7 may include a further different type of medical care information. Still further, a plurality of sets of medical care information to be displayed in the second region R7 is preferably structured such that the type of each set of medical care information and items belonging to each set of medical care information can be set arbitrarily. In each of the embodiments described above, a plurality of sets of medical care information to be displayed in the second region R7 is preferably structured such that the display format, such as simplified display or normal display, is made selectable arbitrarily based on a user operation or a predetermined setting for each type of medical care information.

Still further, in the embodiments described above, a new display date condition may be added to the first to the third display date conditions or any display condition may be omitted from the first to the third display date conditions. As for the display date condition selection method, anymethod that receives a user selection, such as button, pull-down menu, or the like, may be employed. Further, in the embodiments described above, the function that makes a plurality of display date conditions selectable, such as the first to the third display conditions, may be omitted and the display dates may be set according to a single display date condition. Further, the display date condition may be a condition in which display dates are set in the future and past directions from the display reference date.

Further, in each of the embodiments described above, the medical care information display control apparatus 1 may be of a configuration without the display days determination unit 15 and the second region display control unit 14 may perform the second region display control processing as in the manner described above based on a predetermined number of display days. Still further, the processing steps S06 and S07 may be performed in reverse order. Also, the processing steps S08 and S09 may be performed in reverse order.

In the embodiments described above, the medical care information obtaining processing shown in S01 may be accomplished by one operation or through a plurality of operations. Further, the medical care information obtaining processing shown in S01 may be performed at any arbitrary timing prior to the processing of determining display dates in S05. Still further, the processing to obtain medical care information may be performed for a required number of times in parallel with the processing to determine display dates in S05.

As for each index in each of the embodiments described above, any index, such as a point, line, polygon, or the like, may be used as long as it can be identifiably displayed.

It should be appreciated that the present invention is not limited to the embodiments described above, and a part or whole of the components of the medical care information display control apparatus 1 may be configured by one workstation or by more than one workstation, a server, and a storage device connected through a network. Each of the equipment is controlled by a program that performs medical care information display of the present specification installed from a recording medium, such as a CD-ROM or the like. The program may be a program installed after being downloaded from a storage unit of a server connected through a network, such as the Internet.

Each of the embodiments described above illustrates only one aspect of the present invention and modifications and applications may be made arbitrarily without departing from the spirit of the present invention.

## Claims

1. A medical care information display control apparatus, comprising:
a first region display control unit (11) that displays a first region (R2) representing a time axis on a display screen;
a reference date identification unit (12) that receives an input of a position in the displayed first region (R2) and identifies a display reference date according to the received position;
a medical care information obtaining unit (13) that obtains a plurality of sets of medical care information of a display target person which includes a plurality of sets of medical care data associated with a plurality of medical care data dates respectively; and
a second region display control unit (14) that obtains a number of display days of a second region (R7) in which the obtained plurality of sets of medical care information is displayed in time series on a daily basis, determines the plurality of medical care data dates as a plurality of display dates to be displayed in the second region (R7) in the order of closeness to the display reference date in a time axis direction such that the number of display days is satisfied based on the obtained plurality of medical care data dates and the number of display days, and displays the second region (R7) on a display screen based on the determined plurality of display dates and each set of medical care data corresponding to each display date.

2. The medical care information display control apparatus as claimed in claim 1, **characterized in that** the apparatus further comprises a display days determination unit (15) that determines the number of display days according to the size of the second region (R7), such that the greater the size of the second region (R7), the greater the number of display days.

3. The medical care information display control apparatus as claimed in claim 1 or 2, **characterized in that** the medical care information obtaining unit (13) obtains two or more types of medical care information arbitrarily selected from the group consisting of image inspection information, sample test information, medication information, and biological information.

4. The medical care information display control apparatus as claimed in any of claims 1 to 3, **characterized in that** the first region display control unit (11) identifiably displays an index that indicates the plurality of display dates of the second region (R7) in the first region (R2).

5. The medical care information display control apparatus as claimed in claim 4, **characterized in that** the first region display control unit (11) identifiably displays an index that indicates a display period of the second region (R7) with the oldest date of the plurality of display dates as the start date and the latest date of the plurality of display dates as the end date in the first region (R2).

6. The medical care information display control apparatus as claimed in claim 5, **characterized in that**:
the reference date identification unit (12) selects, based on the received position and the position of the index that indicates the display period, any one of a first display date condition in which the plurality of display dates is determined only before the identified display reference date, a second display date condition in which the plurality of display dates is determined only after the display reference date, and a third display condition in which the plurality of display dates is determined both before and after the display reference date; and
the second region display control unit (14) determines the plurality of display dates based on the selected one display date condition.

7. The medical care information display control apparatus as claimed in any of claims 1 to 6, **characterized in that** the first region display control unit (11) further displays an index that identifiably indicates presence or absence of the medical care data along the time axis in the first region (R2).

8. The medical care information display control apparatus as claimed in any of claims 1 to 7, **characterized in that** the second region display control unit (14) determines the plurality of medical care data dates as a plurality of display dates to be displayed in the second region (R7) in the order of closeness to the display reference date in a time axis direction such that the number of display days is satisfied based on a plurality of medical care data dates of a given set of medical care information of the plurality of sets of medical care information and the number of display days, and displays the second region (R7) on a display screen based on the determined plurality of display dates and each set of medical care data corresponding to each display date.

9. A medical care information display control method that operates a medical care information display control apparatus, the method comprising:
a first region display step that displays a first region (R2) representing a time axis on a display screen;
a reference date identification step that receives an input of a position in the displayed first region (R2) and identifies a display reference date according to the received position;
a medical care information obtaining step that obtains a plurality of sets of medical care information of a display target person which includes a plurality of sets of medical care data associated with a plurality of medical care data dates respectively; and
a second region display control step that obtains a number of display days of a second region (R7) in which the obtained plurality of sets of medical care information is displayed in time series on a daily basis, determines the plurality of medical care data dates as a plurality of display dates to be displayed in the second region (R7) in the order of closeness to the display reference date in a time axis direction such that the number of display days is satisfied based on the obtained plurality of medical care data dates and the number of display days, and displays the second region (R7) on a display screen based on the determined plurality of display dates and each set of medical care data corresponding to each display date.

10. A computer readable recording medium on which is recorded a medical care information display control program that causes a computer to function as:
a first region display control unit (11) that displays a first region (R2) representing a time axis on a display screen;
a reference date identification unit (12) that receives an input of a position in the displayed first region (R2) and identifies a display reference date according to the received position;
a medical care information obtaining unit (13) that obtains a plurality of sets of medical care information of a display target person which includes a plurality of sets of medical care data associated with a plurality of medical care data dates respectively; and
a second region display control unit (14) that obtains a number of display days of a second region (R7) in which the obtained plurality of sets of medical care information is displayed in time series on a daily basis, determines the plurality of medical care data dates as a plurality of display dates to be displayed in the second region (R7) in the order of closeness to the display reference date in a time axis direction such that the number of display days is satisfied based on the obtained plurality of medical care data dates and the number of display days, and displays the second region (R7) on a display screen based on the determined plurality of display dates and each set of medical care data corresponding to each display date.
